# EUROPÄISCHE PATENTSCHRIFT

(11) **EP 1 974 051 B1**
(45) Veröffentlichungstag und Bekanntmachung des Hinweises auf die Patenterteilung: **28.07.2010**
(21) Anmeldenummer: 06829780.3
(22) Anmeldetag: 20.12.2006
(51) Int. Cl.: C12Q 1/25, C12Q 1/32, C12Q 1/527

(54) **VERFAHREN UND MITTEL ZUR ENZYMATISCHEN BESTIMMUNG VON ACETAT**
PROCESS AND MEANS FOR ENZYMATIC DETERMINATION OF ACETATE
PROCEDE ET AGENT DE DOSAGE ENZYMATIQUE D'ACETATE

(30) Priorität: 17.01.2006 DE 102006002165
(43) Veröffentlichungstag der Anmeldung: 01.10.2008
(73) Patentinhaber: Merck Patent GmbH, 64293 Darmstadt (DE)
(72) Erfinder: VORMBROCK, Rolf, 64289 Darmstadt (DE)
(86) Internationale Anmeldenummer: PCT/EP2006/012299
(87) Internationale Veröffentlichungsnummer: WO 2007/087882

(56) Entgegenhaltungen:
- WO-A-01/73114
- WO-A-02/094949
- US-A1- 4 152 116
- US-B1- 6 200 773
- US-B2- 6 428 972
- BOEHRINGER MANNHEIM/ R-BIOPHARM: "Acetic acid - Product information" INTERNET ARTICLE, [Online] 13. Dezember 2005 (2005-12-13), XP002423048 Gefunden im Internet: URL:http://web.archive.org/web/20051213213 004/http://www.r-biopharm.de/> [gefunden am 2007-03-02]

## Beschreibung

Die Erfindung betrifft ein Verfahren und ein Mittel zum enzymatischen Nachweis von Acetat. Das erfindungsgemäße Verfahren ermöglicht erstmals die reflektometrische Detektion mittels eines Teststreifens.

Der Nachweis von Acetat spielt auf vielen Gebieten eine wichtige Rolle, wie z.B. in der Industrie zur Kontrolle von Fermentationsprozessen oder zur Bestimmung der Reinheit von Reagenzien, in der Lebensmittelindustrie zur Bestimmung des Acetatgehaltes von Lebensmitteln und Getränken oder in bei der Weinherstellung.

Im Stand der Technik sind viele Methoden zum Nachweis von Acetat bekannt. Teilweise ist für die Durchführung dieser Methoden ein großer technischer Aufwand nötig, zum Beispiel in der Weinanalyse bei der Bestimmung der flüchtigen Säuren, die ein Qualitätsmerkmal darstellen und deren Hauptbestandteil die Essigsäure ist. Daher werden in der Regel enzymatische Nachweismethoden bevorzugt, da sie schnell und einfach durchgeführt werden können. Diese Methoden werden nasschemisch durchgeführt. Ein Überblick über die wichtigsten Methoden zum Nachweis von Acetat findet sich in H.U. Bergmeyer (ed.), Methods of Enzymatic Analysis (3rd Edition, Weinheim 1984, Vol. 6, Seiten 628-645).

Eine von der Firma Boehringer Mannheim angebotene Testmethode umfasst ein Verfahren zur Bestimmung des Acetatgehalts einer Probe, umfassend folgende Verfahrensschritte:
- Bereitstellung einer Probe deren Acetatgehalt bestimmt werden soll,
- Verdünnen und Einstellen des pH-Wertes der verdünnten Probelösung mit einer Pufferlösung, die neben der Puffersubstanz zumindest Äpfelsäure enthält,
- Kontaktieren der Probe mit ATP, Coenzym A, NAD, Malatdehydrogenase, Citratsynthase und Acetyl CoA Synthase,
- Inkubation,
- Bestimmung des Acetatgehalts auf Bsis der NADH Bildung (photochemisch durch Zunahme der Lichtabsorption bei 340, 334 oder 365 nm) [Boehringer Mannheim/R-Biopharm: Acetic acid - Product information" Internet Article, Online 13. Dezember 2005 (2005 - 12 - 13), (URL:http://web.archive.org/web/20051213213 004/http://www.r-biopharm.de/)]

Aus WO 01/73114 A1 ist lediglich ein colorimetrischer Nachweis von NADH aus stark gefärbten Proben, wie z. B. Blut, bekannt, wobei NADH durch eine Malatdehydrogenase katalysierte Reaktion gebildet wird, mittels eines Formazankomplexes nach Reaktion aus Tetrazolium und Phenazinmethosulfat. In US 6,428,972 B2 wird die gleiche Methode beschrieben, wobei jedoch das NAD nach abgelaufener Farbreaktion wieder für die Enzymkatalyse zur Verfügung steht. Beide Literaturstellen befassen sich lediglich mit dem colorimetrischen Nachweis von NADH.

Wie aus dem vorhergehenden zu entnehmen ist, handelt es sich bei der derzeit gängigsten Methode zur Bestimmung von Acetat um eine nasschemische, photometrische Methode unter Verwendung von Acetyl-CoA Synthetase (beschrieben bei H.-O. Beutler [Acetate, Determination with Acetyl-CoA Synthase, in H.U. Bergmeyer (ed.), Methods of Enzymatic Analysis, 3rd Edition, Weinheim 1984, Vol. 6 p 639 - 645]). Der photometrische Nachweis basiert auf der Detektion von NADH.

Da ein photochemischer Nachweis sowohl apparativ aufwendig ist und zumeist stark von der Eigenfärbung der Probe abhängt, wäre es wünschens wert einen Nachweis von Acetat durchführen zu können, bei dem die Detektion reflektometrisch über ein stark gefärbtes Produkt erfolgt.

Aufgabe der vorliegenden Erfindung war es daher, einen Nachweis von Acetat zur Verfügung zu stellen, der schneller und einfacher durchzuführen als die titrimetrische Bestimmung der flüchtigen Säuren oder die bekannte photometrische Methode unter Verwendung von Acetyl-CoA Synthase.

Es wurde gefunden, dass die bekannte photometrische Methode unter Verwendung von Acetyl-CoA Synthase nicht direkt mit einer Farbreaktion gekoppelt werden kann, die das gebildete NADH z.B. unter Reduktion eines Tetrazoliumsalzes zu einem gefärbten Formazan umsetzt. Bei einer solchen Reaktion wird nämlich nicht nur das durch die Acetatbestimmung gebildete NADH umgesetzt. Vielmehr wird unabhängig von der Acetatkonzentration so viel Formazan gebildet, bis eine der im Test eingesetzten Komponenten Tetrazoliumsalz oder Malat vollständig umgesetzt ist. Somit ist eine quantitative Bestimmung von Acetat auf diese Weise nicht möglich.

Es wurde weiterhin gefunden, dass eine reflektometrische Bestimmung von Acetat möglich ist, wenn die Acetatbestimmung in zwei Schritten durchgeführt wird, wobei in einem ersten Schritt auf einem Teststreifen eine Reaktion gemäß der Methode von H.-O. Beutler [Acetate, Determination with Acetyl-CoA Synthase in H.U. Bergmeyer (ed.), Methods of Enzymatic Analysis, 3rd Edition, Weinheim 1984, Vol. 6, S 639 - 645] durchgeführt wird. Anschließend wird in einem zweiten Schritt die Konzentration des entstandenen NADH auf einem Teststreifen mittels Phenazinmethosulfat oder einem Derivat davon unter Reduktion eines Tetrazoliumsalzes zu einem gefärbten Formazan bestimmt. Bei dieser Vorgehensweise wird der Einfluss der Malat-Dehydrogenase aus dem ersten Reaktionsschritt so weit herabgesetzt, dass er nicht zu einer Verfälschung des Ergebnisses führt. Nach dem Ende der Reaktion auf dem Teststreifen wird die gebildete Farbe des Formazans reflektometrisch als Remission gemessen. Über eine Kalibrationskurve kann hieraus die Konzentration des Acetats in der Probe berechnet werden.

Gegenstand der vorliegenden Erfindung ist daher ein Verfahren zur Bestimmung des Acetatgehalts einer Probe, umfassend zumindest folgende Verfahrensschritte:
a) Bereitstellung einer Probe deren Acetatgehalt bestimmt werden soll
b) Verdünnen und Einstellen des pH-Wertes der verdünnten Probelösung mit einer Pufferlösung, die Äpfelsäure enthält
c) Kontaktieren der verdünnten Probe mit einem Teststreifen, der zumindest ein Tetrazoliumsalz, ATP, Coenzym A, NAD, Malatdehydrogenase, Citratsynthase und Acetyl CoA Synthetase enthält;
d) Inkubation des Teststreifens aus Schritt c)
e) Kontaktieren des Teststreifens aus Schritt d) mit einer flüssigen Reagenzzusammenstellung, die zumindest Phenazirimethosulfat oder ein Derivat davon enthält und einen pH-Wert im Bereich zwischen 2 und 6 aufweist,
f) Inkubation des Teststreifens aus Schritt e)
g) Bestimmung des Acetatgehalts anhand der entstehenden Farbentwicklung

In einer besonderen Ausführungsform wird in Schritt b) ein Puffer verwendet, der als Puffersubstanz Triethanolamin verwendet
In einer weiteren bevorzugten Ausführungsform kann in Schritt c) ein Teststreifen verwendet, der als Tetrazoliumsalz Nitroblautetrazoliumchlorid enthält.

In einer anderen erfindungsgemäßen Ausführungsform ist die in Schritt a) bereitgestellte Probe ein Getränk.

In einer weiteren bevorzugten Ausführungsform hat die Reagenzzusammenstellung aus Schritt e) einen pH-Wert zwischen 2,5 und 5,5.

In einer bevorzugten Ausführungsform erfolgt die Bestimmung des Acetatgehaltes in Schritt g) reflektometrisch.

Gegenstand der vorliegenden Erfindung ist auch ein Test-Kit zur Bestimmung des Acetatgehalts einer Probe, zumindest enthaltend
a) Eine Pufferlösung, die neben der eigentlichen Puffersubstanz Äpfelsäure enthält.
b) einen Teststreifen, der zumindest ein Tetrazoliumsalz, ATP, Coenzym A, NAD, Malatdehydrogenase, Citratsynthase und Acetyl CoA Synthetase enthält;
c) eine flüssige Reagenzzusammenstellung, die zumindest Phenazinmethosulfat oder ein Derivat davon enthält und einen pH-Wert im Bereich zwischen 2 und 6 aufweist.

In einer bevorzugten Ausführungsform enthalten Pufferlösung, Teststreifen und Reagenzzusammenstellungen unabhängig voneinander Puffer und Stabilisatoren.

In einer bevorzugten Ausführungsform enthält der Teststreifen als Tetrazoliumsalz Nitroblautetrazoliumchlorid.

In einer anderen bevorzugten Ausführungsform hat die Reagenzzusammenstellung aus Schritt e) einen pH-Wert zwischen 2,5 und 5,5.

In der Beschreibung der Erfindung werden die folgenden Abkürzungen verwendet:

| | |
|---|---|
| NAD | Nicotinamidadenindinucleotid (oxidierte Form) |
| NADH | Nicotinamidadenindinucleotid (reduzierte Form) |
| NBT | Nitroblautetrazoliumchlorid |

Erfindungsgemäß ist eine Probe ist typischerweise flüssig. Sie kann beispielsweise eine Lösung oder Suspension sein, die in der Regel als vorwiegendes Lösungsmittel Wasser oder eine Pufferlösung enthält. Feste Proben werden daher zunächst in Wasser oder einer Pufferlösung gelöst, suspendiert oder damit extrahiert. Beispiele für Proben sind Getränke, wie Frucht- oder Gemüsesäfte, alkoholische Getränke, insbesondere Weine, oder Blut. Je nach Beschaffenheit der Probe und deren Acetatgehalt kann es vorteilhaft sein, die Probe vor der Durchführung der erfindungsgemäßen Bestimmung mit Wasser zu verdünnen.

In jüngerer Zeit hat die Analyse mit festen, saugfähigen Trägem, den sogenannten Teststäbchen oder Teststreifen, zunehmend an Bedeutung gewonnen. Zu den wesentlichen Vorteilen dieser trockenchemischen Verfahren gehören insbesondere die einfache Handhabung sowie die aufgrund der geringen Reagenzmengen unproblematische Entsorgung. Alle oder der größte Teil der für die Nachweisreaktion notwendigen Reagenzien sind dabei in entsprechenden Schichten eines festen, saugfähigen oder quellbaren Trägers eingebettet, auf den die Probe aufgebracht wird. Nach Kontakt der Reaktionszone mit der Probe läuft die Nachweisreaktion ab. Die gebildete Farbe ist ein Maß für die Menge des zu bestimmenden Analyten und kann visuell, d.h. halbquantitativ, bzw. quantitativ mit einfachen Reflektometern ausgewertet werden.

Als saugfähige Träger für die Teststreifen des erfindungsgemäßen Test-Kits können alle Materialien verwendet werden, die üblicherweise für solche trockenchemischen Tests im Gebrauch sind. Am weitesten verbreitet ist die Verwendung von Filterpapier, jedoch können auch andere saugfähige Cellulose- oder Kunststoffprodukte eingesetzt werden.

Die saugfähigen Träger werden in bekannter Weise mit Tränklösungen imprägniert. Die getränkten und getrockneten Träger können geeignet zugeschnitten und in bekannter Weise zur Herstellung der Teststreifen auf Trägerfolien aufgeklebt bzw. aufgesiegelt werden.

Zumeist bedeckt der saugfähige Träger, auf den die Nachweisreagenzien aufgebracht sind, nicht den gesamten Teststreifen, sondern lediglich eine Zone des Teststreifens. Auf diese Weise ist es möglich, nicht nur eine Zone mit einer Zusammensetzung von Nachweisreagenzien, sondern mehrere Zonen mit gleicher oder unterschiedlichen Zusammensetzungen auf einem Teststreifen zu kombinieren. Erfindungsgemäß wird daher der Bereich des Teststreifens, auf den die für den Nachweis eines Analyten oder zur Erstellung eines Leerwerts notwendigen Reagenzien auf einen saugfähigen Träger aufgebracht sind, als Zone bezeichnet.

Acetat bedeutet erfindungsgemäß Essigsäure sowie Salze der Essigsäure.

ATP bedeutet erfindungsgemäß Adenosintriphosphat sowie Salze dieser Verbindung, die von den erfindungsgemäß eingesetzten Enzymen in gleicher Weise umgesetzt werden können, wie z.B. Adenosintriphosphat di-Natriumsalz.

Coenzym A bedeutet erfindungsgemäß Coenzym A Salze dieser Verbindung, die von den erfindungsgemäß eingesetzten Enzymen in gleicher Weise umgesetzt werden können, wie z.B. Coenzym A tri-Lithiumsalz.

Phenazinmethosulfat bedeutet erfindungsgemäß 5-Methylphenaziniummethylsulfat oder Derivate davon, wie z.B. 1-Methoxy-5-Methylphenaziniummethylsulfat.

Die erfindungsgemäße Bestimmung des Acetatgehalts erfolgt in zwei Schritten. In einem ersten Schritt wird Acetat auf einem Teststreifen entsprechend der Methode von H.-O. Beutler [Acetate, Determination with Acetyl-CoA Synthase in H.U. Bergmeyer (ed.), Methods of Enzymatic Analysis, 3rd Edition, Weinheim 1984, Vol. 6, S. 639 - 645] umgesetzt. Das dabei entstehende NADH wird dann in einem zweiten Schritt verbraucht, indem ein auf dem Teststreifen befindliches Tetrazoliumsalz durch Zugabe von Phenazinmethosulfat oder Derivaten dieser Verbindung zu einem gefärbten Formazan umgesetzt wird, wobei diese Reaktion bevorzugt bei pH-Werten unter pH 6,0 durchgeführt wird.

Die für die Probenverdünnung eingesetzte Pufferlösung enthält zumindest eine Puffersubstanz und Äpfelsäure. Bevorzugte Konzentrationsbereiche dieser Komponenten in den erfindungsgemäßen Lösungen liegen im Bereich von:

| | |
|---|---|
| Triethanolamin und | 0,2 bis 1,6 mol/l |
| und | |
| L-Äpfelsäure | 20 bis 100 mmol/l. |

Besonders bevorzugte Konzentrationen sind:

| | | |
|---|---|---|
| 1,0 bis 1,5 | mol/l | Triethanolamin |
| 50 bis 80 | mmol/l | L-Äpfelsäure |

Der im ersten Reaktionsschritt eingesetzte Teststreifen enthält zumindest ein Tetrazoliumsalz, ATP, Coenzym A, NAD, Malatdehydrogenase, Citratsynthase und Acetyl CoA Synthetase.

Dazu wird ein Teststreifen nach bekannten Methoden mit einer oder mehreren Tränklösungen getränkt, die die entsprechenden Substanzen enthalten.

Die Konzentrationen der einzelnen Bestandteile in den Tränklösungen entsprechen den Konzentrationen, die auch für einen nasschemischen Acetatnachweis nach dem Stand der Technik verwendet werden können.

Bevorzugte Konzentrationsbereiche sind

| | | |
|---|---|---|
| 1,0 bis 5,0 | mmol/l | Magnesiumchlorid oder -sulfat |
| 10 bis 25 | mmol/l | Adenosintriphosphat di-Natriumsalz |
| 10 bis 30 | mmol/l | Nicotinamid-adenin-dinucleotid |
| 0,8 bis 1,5 | mmol/l | Coenzym A tri-Lithiumsalz |
| 20 bis 100 | U/ml | Malatdehydrogenase |
| 5 bis 25 | U/ml | Citratsynthase |
| 1,0 bis 5,0 | U/ml | Acetyl-CoA Synthetase |

Besonders bevorzugte Konzentrationen sind:

| | | |
|---|---|---|
| 2,0 | mmol/l | Magnesiumchlorid |
| 15 | mmol/l | Adenosintriphosphat di-Natriumsalz |
| 12 | mmol/l | Nicotinamid-adenin-dinucleotid |
| 1,1 | mmol/l | Coenzym A tri-Lithiumsalz |
| 43 | U/ml | Malatdehydrogenase |
| 14 | U/ml | Citratsynthase |
| 1,6 | U/ml | Acetyl-CoA Synthetase |

Das Tetrazoliumsalz ist in den Tränklösungen typischerweise in einer Konzentration zwischen 0,5 bis 5,0 g/l, bevorzugt zwischen 500 mg und 2 g/l enthalten. Geeignete Tetrazoliumsalze sind NBT oder MTT (3-(4,5-Dimethylthiazol-2-yl)-2,5-diphenyl-2-tetrazoliumbromid).

In der Regel sind die Tränklösungen wässrige Lösungen, die einen Anteil eines oder mehrerer Alkohole, wie Methanol oder bevorzugt Ethanol enthalten können. Weiterhin enthalten die Tränklösungen typischerweise Salze, Puffer und/oder Stabilisatoren. Der Fachmann auf dem Gebiet der Teststreifenherstellung kennt die dafür geeigneten Substanzen.

Für den erfindungsgemäßen Teststreifen hat die Tränklösung bevorzugt einen pH-Wert zwischen 7,0 und 7,5, besonders bevorzugt 7,2. Dieser pH-Wert kann beispielsweise mit einem Triethanolamin-Puffer oder einem BIS-TRIS-Propan - Puffer erzeugt werden.

Der Teststreifen kann z.B. durch kurzes Eintauchen in die verdünnte Probe oder Auftropfen der verdünnten Probe auf den Teststreifen mit der Probe in Kontakt gebracht werden.

Um eine möglichst vollständige Umsetzung des Acetats in der Probe zu gewährleisten, wird die Probe auf dem Teststreifen inkubiert. Die Inkubationszeit liegt typischerweise zwischen 30 Sekunden und 20 Minuten, bevorzugt bei 5 bis 15 Minuten. Bevorzugt erfolgt die Inkubation bei Temperaturen zwischen 18 und 35 °C, besonders bevorzugt bei Raumtemperatur.

Anschließend wird erfindungsgemäß in einem zweiten Schritt (Verfahrensschritte e) bis f) des erfindungsgemäßen Verfahrens) die Konzentration des entstandenen NADH bestimmt. Dies erfolgt durch Inkontaktbringen des Teststreifens aus Verfahrensschritt d) mit einer Reagenzkombination, die zumindest Phenazinmethosulfat oder ein geeignetes Derivat davon enthält.

Diese Reagenzzusammenstellung liegt in flüssiger Form, typischerweise als Lösung, vor. Bevorzugte Konzentrationsbereiche von Phenazinmethosulfat oder von einem geeigneten Derivat liegen zwischen 25 und 400 µg/ml, besonders bevorzugt zwischen 60 und 150 µg/ml. In der Regel enthält die Reagenzzusammenstellung zudem Puffer und/oder Stabilisatoren. Der pH-Bereich der Reagenzzusammenstellung ist zwischen pH 2 und pH 6, besonders bevorzugt zwischen pH 3 und 5. Geeignete Puffer zur Einstellung dieses Bereiches sind Puffersubstanzen mit einem pKₛ-Wert im Bereich von 2,0 bis 6,5, vorzugsweise Salze der Citronensäure.

Um eine möglichst vollständige Umsetzung des NADH zu gewährleisten, wird nach Inkontaktbringen des Teststreifens mit der Reagenzzusammenstellung inkubiert. Die Inkubationszeit liegt typischerweise zwischen 30 Sekunden und 10 Minuten, bevorzugt bei ca. 5 Minuten. Bevorzugt erfolgt die Inkubation bei Temperaturen zwischen 18 und 35 °C, besonders bevorzugt bei Raumtemperatur.

Nach dem Ende der Reaktion wird die Intensität der Farbe des Formazans bestimmt. Dies kann visuell erfolgen, z.B. halbquantitativ durch einen Vergleich mit einer Farbkarte oder quantitativ z.B. mittels eines Reflektometers. Über eine Kalibrationskurve kann hieraus die Konzentration des Acetats in der Probe berechnet werden.

Gegenstand der vorliegenden Erfindung ist auch ein Test-Kit zur Durchführung des erfindungsgemäßen Verfahrens.

Der Test-Kit enthält zumindest
a) Eine Pufferlösung zur Verdünnung der Probe und Einstellung des pH-Wertes, die zumindest eine Puffersubstanz und Äpfelsäure enthält;
b) Einen Teststreifen, der zumindest ein Tetrazoliumsalz, ATP, Coenzym A, NAD, Malatdehydrogenase, Citratsynthase und Acetyl CoA Synthetase enthält;
c) eine flüssige Reagenzzusammenstellung, die zumindest eine Puffersubstanz zur Einstellung des pH-Wertes im Bereich zwischen 2 und 6 und Phenazinmethosutfat oder ein Derivat davon enthält.

Bevorzugt enthält der Test-Kit weiterhin eine Beschreibung zur Durchführung des erfindungsgemäßen Verfahrens.
Bezüglich der Zusammensetzung der Reagenzzusammenstellungen und der bevorzugten Ausführungsformen wird auf die Ausführungen bei der Beschreibung des erfindungsgemäßen Verfahrens verwiesen.

Durch das zur Verfügung gestellte erfindungsgemäße Nachweisverfahren ist es möglich, Essigsäurekonzentrationen von 40 - 400 mg/l in Form von Acetat nachzuweisen. Höhere Konzentrationen können nach entsprechender Verdünnung mit Wasser bestimmt werden.

Somit bieten das erfindungsgemäße Verfahren und der erfindungsgemäße Test-Kit eine einfache, schnelle und empfindliche Methode zum enzymatischen Nachweis von Acetat. Durch die einfache Verfahrensdurchführung mithilfe eines Teststreifens kann der Nachweis ohne apparativen Aufwand und ohne Handhabung von giftigen Chemikalien auch von ungeübtem Personal durchgeführt werden.

Auch ohne weitere Ausführungen wird davon ausgegangen, daß ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiel

### Bestimmung von Essigsäure in Wein

Es werden Teststreifen mit zwei Papierzonen für die Essigsäurebestimmung hergestellt, die im ersten Schritt mit einer Lösung von NitroblauTetrazoliumchlorid in Triethanolamin-Puffer pH 7,2 getränkt werden. Die Lösung ist folgendermaßen zusammengesetzt:

| | | |
|---|---|---|
| 45 | mmol/l | Triethanolamin-Puffer pH 7,2 |
| 60 | g/l | Ethanol |
| 1 | g/l | Nitroblautetrazoliumchlorid Füllstoffe und Stabilisatoren |

Anschließend wird auf beide Zonen eine Lösung folgender Zusammensetzung aufgetragen:

| | | |
|---|---|---|
| 2,0 | mmol/l | Magnesiumchlorid Hexahydrat |
| 15 | mmol/l | Adenosintriphosphat di-Natriumsalz |
| 12 | mmol/l | Nicotinamid-adenin-dinucleotid |
| 1,1 | mmol/l | Coenzym A tri-Lithiumsalz |
| 43 | U/ml | Malatdehydrogenase |
| 14 | U/ml | Citratsynthase |
| 1,6 | U/ml | Acetyl-CoA Synthetase Füllstoffe und Stabilisatoren |

Nach dem Trocknen werden diese Teststreifen bei 2°C bis 8°C trocken gelagert.

Die Bestimmung wird wie folgt durchgeführt:
1,0 ml einer vorverdünnten Probe wird mit 1,0 ml Pufferlösung (60 mmol/l Äpfelsäure in 1,4 mol/l Triethanolaminpuffer pH 8,0) versetzt und mit 8,0 ml Wasser verdünnt. In diese Lösung wird ein Teststäbchen getaucht. Überschüssige Flüssigkeit wird vom Teststreifen entfernt, und der Teststreifen wird 10 Minuten bei Raumtemperatur inkubiert.

Anschließend wird auf jede Papierzone je ein Tropfen einer Lösung von 1-Methoxy-5-Methylphenazinium Methylsulfat in einer Citrat-Pufferlösung pH 3,5 aufgetropft und nach weiteren 5 Minuten Reaktionszeit wird die gebildete Färbung der Papierzonen reflektometrisch gemessen. Über eine Kalibrationskurve wird die Essigsäurekonzentration berechnet.

## Patentansprüche

1. Verfahren zur Bestimmung des Acetatgehalts einer Probe, umfassend zumindest folgende Verfahrensschritte:
a) Bereitstellung einer Probe deren Acetatgehalt bestimmt werden soll
b) Verdünnen und Einstellen des pH-Wertes der verdünnten Probelösung mit einer Pufferlösung, die neben der Puffersubstanz zumindest Äpfelsäure enthält
c) Kontaktieren der Probe mit einem Teststreifen, der zumindest ein Tetrazoliumsalz, ATP, Coenzym A, NAD, Malatdehydrogenase, Citratsynthase und Acetyl CoA Synthetase enthält;
d) Inkubation des Teststreifens aus Schritt c)
e) Kontaktieren des Teststreifens aus Schritt d) mit einer flüssigen Reagenzzusammenstellung, die zumindest Phenazinmethosulfat oder ein Derivat davon enthält und einen pH-Wert im Bereich zwischen 2 und 6 aufweist,
f) Inkubation des Teststreifens aus Schritt e)
g) Bestimmung des Acetatgehalts anhand der entstehenden Farbentwicklung.

2. Verfahren nach Anspruch 1, **dadurch gekennzeichnet, dass** in Schritt c) ein Teststreifen verwendet wird, der als Tetrazoliumsalz Nitroblautetrazoliumchlorid enthält.

3. Verfahren nach Anspruch 1 oder 2, **dadurch gekennzeichnet, dass** die in Schritt a) bereitgestellte Probe ein Getränk ist.

4. Verfahren nach einem oder mehreren der Ansprüche 1 bis 3, **dadurch gekennzeichnet, dass** der Nachweis des Acetatgehaltes in Schritt g) reflektometrisch erfolgt.

5. Test-Kit zur Bestimmung des Acetatgehalts einer Probe, zumindest enthaltend
a) Eine Pufferlösung zur Verdünnung der Probe und Einstellung des pH-Wertes, die zumindest eine Puffersubstanz und Äpfelsäure enthält;
b) Einen Teststreifen, der zumindest ein Tetrazoliumsalz, ATP, Coenzym A, NAD, Malatdehydrogenase, Citratsynthase und Acetyl CoA Synthetase enthält;
c) eine flüssige Reagenzzusammenstellung, die zumindest Phenazinmethosulfat oder ein Derivat davon enthält und einen pH-Wert im Bereich zwischen 2 und 6 aufweist.

6. Test-Kit nach Anspruch 5, **dadurch gekennzeichnet, dass** Pufferlösung, Teststreifen und Reagenzzusammenstellungen unabhängig voneinander Puffer und Stabilisatoren enthalten.

7. Test-Kit nach Anspruch 5 oder 6, **dadurch gekennzeichnet, dass** der Teststreifen als Tetrazoliumsalz Nitroblautetrazoliumchlorid enthält.

## Claims

1. Method for the determination of the acetate content of a sample, comprising at least the following method steps:
a) provision of a sample whose acetate content is to be determined
b) dilution and setting of the pH of the diluted sample solution by means of a buffer solution which, besides the buffer substance, comprises at least malic acid
c) bringing the sample into contact with a test strip which comprises at least a tetrazolium salt, ATP, coenzyme A, NAD, malate dehydrogenase, citrate synthase and acetyl CoA synthetase
d) incubation of the test strip from step c)
e) bringing the test strip from step d) into contact with a liquid reagent composition which comprises at least phenazine methosulfate or a derivative thereof and has a pH in the range between 2 and 6
f) incubation of the test strip from step e)
g) determination of the acetate content with reference to the colour development forming.

2. Method according to Claim 1, **characterised in that** a test strip which comprises nitro blue tetrazolium chloride as tetrazolium salt is used in step c).

3. Method according to Claim 1 or 2, **characterised in that** the sample provided in step a) is a beverage.

4. Method according to one or more of Claims 1 to 3, **characterised in that** the determination of the acetate content in step g) is carried out reflectometrically.

5. Test kit for the determination of the acetate content of a sample, at least comprising
a) a buffer solution for dilution of the sample and setting of the pH which comprises at least a buffer substance and malic acid;
b) a test strip which comprises at least a tetrazolium salt, ATP, coenzyme A, NAD, malate dehydrogenase, citrate synthase and acetyl CoA synthetase;
c) a liquid reagent composition which comprises at least phenazine methosulfate or a derivative thereof and has a pH in the range between 2 and 6.

6. Test kit according to Claim 5, **characterised in that** buffer solution, test strip and reagent composition comprise, independently of one another, buffers and stabilisers.

7. Test kit according to Claim 5 or 6, **characterised in that** the test strip comprises nitro blue tetrazolium chloride as tetrazolium salt.

## Revendications

1. Méthode de détermination de la teneur en acétate d'un échantillon, comprenant au moins les étapes opératoires suivantes :
a) la fourniture d'un échantillon dont on doit déterminer la teneur en acétate
b) la dilution et l'ajustement du pH de la solution diluée de l'échantillon au moyen d'une solution tampon qui comprend, outre la substance tampon, au moins de l'acide malique
c) la mise en contact de l'échantillon avec une bandelette réactive comprenant au moins un sel de tétrazolium, de l'ATP, du coenzyme A, du NAD, de la malate déshydrogénase, de la citrate synthétase et de l'acétyl-CoA synthétase
d) l'incubation de la bandelette réactive issue de c)
e) la mise en contact de la bandelette réactive issue de l'étape d) avec une composition de réactifs liquide comprenant au moins du méthosulfate de phénazine ou un dérivé de celui-ci et ayant un pH dans la plage entre 2 et 6
f) l'incubation de la bandelette réactive issue de l'étape e)
g) la détermination de la teneur en acétate en faisant référence au développement de la couleur qui se forme.

2. Méthode selon la revendication 1, **caractérisée en ce qu'**une bandelette réactive comprenant du chlorure de nitro bleu tétrazolium comme sel de tétrazolium est utilisée dans l'étape c).

3. Méthode selon la revendication 1 ou 2, **caractérisée en ce que** l'échantillon fourni dans l'étape a) est une boisson.

4. Méthode selon l'une ou plusieurs des revendications 1 à 3, **caractérisée en ce que** la détermination de la teneur en acétate dans l'étape g) est réalisée par réflectométrie.

5. Kit de test pour la détermination de la teneur en acétate d'un échantillon, comprenant au moins
a) une solution tampon pour la dilution de l'échantillon et l'ajustement du pH, comprenant au moins une substance tampon et de l'acide malique ;
b) une bandelette réactive comprenant au moins un sel de tétrazolium, de l'ATP, du coenzyme A, du NAD, de la malate déshydrogénase, de la citrate synthétase et de l'acétyl-CoA synthétase ;
c) une composition de réactifs liquide comprenant au moins du méthosulfate de phénazine ou un dérivé de celui-ci et ayant un pH dans la plage entre 2 et 6.

6. Kit de test selon la revendication 5, **caractérisé en ce que** la solution tampon, la bandelette réactive et la composition de réactifs comprennent, indépendamment les unes des autres, des tampons et des agents stabilisants.

7. Kit de test selon la revendication 5 ou 6, **caractérisé en ce que** la bandelette réactive comprend du chlorure de nitro bleu tétrazolium comme sel de tétrazolium.
